# EUROPÄISCHE PATENTSCHRIFT

(11) **EP 0 476 113 B1**
(45) Veröffentlichungstag und Bekanntmachung des Hinweises auf die Patenterteilung: **13.03.1996**
(21) Anmeldenummer: 91907526.7
(22) Anmeldetag: 06.04.1991
(51) Int. Cl.: A61K 31/70, A61K 31/725

(54) **ARZNEIMITTEL ZUR BEHANDLUNG VON HYPERLIPIDÄMIE UND/ODER ATHEROSKLEROSE**
MEDICAMENT FOR TREATING HYPERLIPIDAEMIA AND/OR ATHEROSCLEROSIS
MEDICAMENT POUR LE TRAITEMENT DE L'HYPERLIPEMIE ET/OU DE L'ATHEROSCLEROSE

(30) Priorität: 06.04.1990 DE 4011285
(43) Veröffentlichungstag der Anmeldung: 25.03.1992
(73) Patentinhaber: STEIGERWALD ARZNEIMITTELWERK GMBH, D-64295 Darmstadt (DE)
(72) Erfinder: SCHÄFER, Hans-Ludwig, D-6121 Erbach-Ernsbach (DE); SCHNEIDER, Werner, D-5400 Koblenz (DE)
(74) Vertreter: Rupprecht, Klaus, Dipl.-Ing.
(86) Internationale Anmeldenummer: EP9100654
(87) Internationale Veröffentlichungsnummer: WO9115214

(56) Entgegenhaltungen:
- DD-A- 271 415
- FR-A- 2 103 290
- US-A- 2 370 961
- DERWENT PUBLICATIONS HOST EPOQUE FILE WPIL An 85-008836; & JP-A-59 206 045
- DERWENT PUBLICATIONS HOST EPOQUE FILE WPIL An 79-78149B; & JP-A-54 119 038

## Beschreibung

Die Erfindung bezieht sich auf ein oral verabreichbares Arzneimittel zur Behandlung sowie Prophylaxe von Stoffwechselstörungen, insbesondere zur Beeinflussung des Lipid- und Cholesterinstoffwechsels, also der Hyperlipidämie sowie der Atherosklerose.

Seit längerer Zeit wird in den westlichen Industrieländern ein rapides Ansteigen von Stoffwechselstörungen, insbesondere Störungen des Fettstoffwechsels, beobachtet, deren Hauptursache Überernährung bei gleichzeitiger Bewegungsarmut ist. Es bilden sich allmählich erhöhte Blutspiegel bestimmter Lipide aus, welche das Risiko atherosklerotischer Herz- und peripherer Gefäßerkrankungen erhöhen. Die Fette werden dabei im Kreislauf in Form kleinster Tröpfchen (Chylomikronen), die durch einen Proteinfilm (α- oder β-Globulin) stabilisiert sind, transportiert.

Durch den reichlichen Fleischkonsum kommt es oft zu einem Überangebot an Cholesterin, da dieses Steroid bereits in ausreichender Menge in der menschlichen Leber biosynthetisiert wird. Der natürliche Regulationsmechanismus des Cholesterinspiegels wird bei fettreicher Ernährung gestört, und es kommt zu einer dauernden Erhöhung des Plasmacholesterins. Das schwerlösliche Cholesterin lagert sich u.a. in den Gefäßwandschichten der Aorta, in der Hornhaut und in der Linse des Auges ab. Erhöhte Blutcholesterinspiegel sind mindestens zum Teil verantwortlich für die Entstehung von arteriellen Gefäßsklerosen. Hypercholesterinämie ist eine die Hyperlipidämie stets begleitende Stoffwechselstörung, welche z. B. bei Diabetes mellitus sehr ausgeprägt sein kann.

Die pathogenetisch verschiedenen, symptomatisch ähnlichen Krankheitsbilder der Hypertriglyceridämie = Hyperlipidämie, d.h. die Trübung des Blutserums durch Chylomikronen (neutralfettreiche Tröpfchen mit einem Durchmesser von bis 1nm) und die Hypercholesterinämie, d.h. die Erhöhung des Cholesteringehaltes im Blutplasma auf über 200 mg werden unter den Sammelbegriffen Hyperlipoproteinämie bzw. Hyperlipidämie zusammengefaßt (vgl. Pschyrembel, Klin. Wörterbuch).

Zur Therapie der Hyperlipoproteinämie werden bis heute vorwiegend α-(p-Chlorphenoxy)-Isobuttersäureethylester, Salze von α-(p-Chlorphenoxy)-Isobuttersäure und Nikotinsäure sowie Nikotinsäure-Derivate, künstliche Anionenaustauscher-Harze und zahlreiche Kombinationspräparate verwendet.

Die künstlichen Anionenaustauscher-Harze führen aufgrund ihrer chemischen und physikalischen Eigenschaften häufig zu erheblichen gastrointestinalen Beschwerden. Sie können aufgrund ihrer anionenaustauschenden Eigenschaften die Resorption anderer Arzneimittel sowie natürlicher Mineralien nachteilig beeinflussen.

In der JP-A-59 206 045 ist zwar die Nützlichkeit von Polymeren der Galacturonsäure zur Prophylaxe und Therapie der Hyperlipidämie und der Atherosklerose erwähnt, jedoch ausschließlich in Verbindung mit extrakorporaler Anwendung. Bei dieser Vorgehensweise wird dem Patienten Blut entnommen, das in eine Zellfraktion und Serum - außerhalb des Körpers - getrennt wird. Dabei wird das Serum durch eine Absorptionskolonne geführt, in der insbesondere die Lipoproteine niedriger Dichte (LDL) absorbiert werden. Das Material der Kolonne beinhaltet einen Träger, der mit Polyanionen beladen ist, die auch Polygalacturonsäuren sein können. Beim Durchfluß durch die Kolonne werden die im Serum enthaltenen LDL an die Polygalacturonsäuren gebunden und somit aus dem Serum entnommen. Das auf diese Weise gereinigte Serum wird anschließend mit dem Zellmaterial wieder vermischt und auf den Patienten übertragen.

Gemäß FR-A-2 103 290 sind mit Methanol veresterte faserige Polygalacturonsäureverbindungen mit α-Glykosid 1,4 Bindung angesprochen. Die gewählte wissenschaftliche Beschreibung ist jedoch unzutreffend. Die faserige Modifikation ist, wie der Vorerfinder selbst ausführt, Pektin, das in Wasser nur quellbar, nicht aber löslich ist und deshalb praktisch nicht in therapeutisch wirksamen Dosen verabreicht werden kann.

In der JP-A-54 119 038 werden ebenfalls Pektin sowie hoch- oder niedermolekulares Methoxypektin als Zugabe zu einem Nahrungsmittel in geringen Mengen von 5 bis 10 Gewichtsprozenten bei Pektin beschrieben.

Arzneimittel auf pflanzlicher Basis, die zur Behandlung der besprochenen Stoffwechselstörungen eingesetzt werden können, sind bisher nicht bekannt geworden. Es wurde nun überraschenderweise gefunden, daß die Galacturonsäure hypocholesterinämische Wirkung zeigt. Mit den im folgenden beschriebenen Versuchen wurde nachgewiesen, daß die Einnahme von Galacturonsäure bzw. gewisser Derivate dieser Säure zu einer deutlichen Senkung des Serumcholesterins führt, daß also diese Verbindung bzw. Verbindungsklasse zur Behandlung von Hyperlipidämie und Atherosklerose sehr gut geeignet ist. Die Erfindung richtet sich somit zunächst zum einen auf die Verwendung von Galacturonsäure, insbesondere der α-D Galacturonsäure der allgemeinen Formel 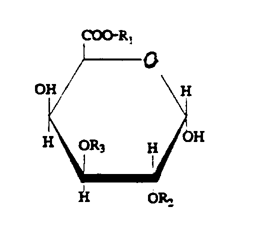
wobei
- R₁: = H,
- R₂: = H und
- R₃: = H,
zur Herstellung eines Arzneimittels zur Prophylaxe und Therapie von Hyperlipidämie und/oder Atherosklerose.

Weiterhin betrifft die Erfindung die Verwendung von Polymeren der Galacturonsäure, insbesondere der α-D Galacturonsäure der allgemeinen Formel, 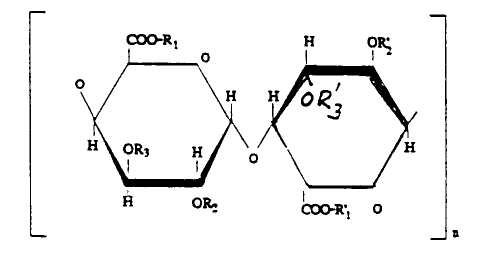
wobei
- R₁: = H R'₁ = H
- R₂: = H R'₂ = H
- R₃: = H R'₃ = H
n eine ganze Zahl ist zu dem in Anspruch 1 angegebenen Zweck.

Auch schließt die Erfindung ein die Verwendung von Methylester der Galacturonsäure und deren Polymere, insbesondere der α-D Galacturonsäure zu dem in Anspruch 1 angegebenen Zweck,
wobei
- R₁: = CH₃ R'₁ = CH₃ oder H,
- R₂: = H R'₂ = H
- R₃: = H R'₃ = H
Auch liegt im Rahmen der Erfindung die Verwendung zu dem in Anspruch 1 angegebenen Zweck von tertiäre oder quartäre Amin-Anionenaustauscher enthaltende Ester, Ether und/oder Acetale der Galacturonsäure, insbesondere der α-D Galacturonsäure sowie deren Polymere, wobei
im Falle der Ester
- R₁: = - CH₂ - R₄ R'1 = R₁ oder H
- R₂: = H R'2 = H
- R₃: = H R'3 = H,
im Falle der Ether
- R₁: = H oder CH₃ R'1 = R₁
- R₂: oder R₃ = - CH₂ - R₄ R'2 = R₂ oder H
R'3 = R₃ oder H,
im Falle der Acetale 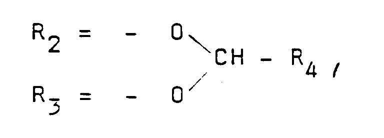
die Amin-Anionenaustauscher in der allgemeinen Formel durch 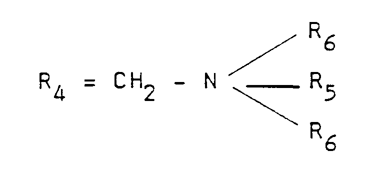
gebildet sind,
und wobei im Falle des tertiären Amins
- R₅: = H
und im Falle des quartären Amins
- R₅: = CH₃ oder
- R₅: = [CH₂] ₘ - CH₃ oder
- R₅: = [CH₂] ₘ CH (OH) - CH₃
- R₆: = [CH₂] ₘ CH₃, wobei
m = eine ganze Zahl von 1 bis 5 ist.

Eine besonders bevorzugte Ausführungsform der Erfindung ergibt sich bei der Verwendung von fermentiertem Pektin zu dem in Anspruch 1 angegebenen Zweck. Das Pektin läßt sich auch durch Fermentation vergleichsweise einfach im wesentlichen zu α-D-Galacturonsäure umformen.

Schließlich bezieht sich die Erfindung auch noch auf ein oral verabreichbares Arzneimittel zur Behandlung von Hyperlipidämie und/oder Atherosklerose. Es ist dadurch gekennzeichnet, daß es als Wirkstoff mindestens eine Verbindung der in Anspruch 1 bis 5 beschriebenen Zusammensetzung enthält.

Im Gegensatz zu der rein synthetischen Medikamenten gehören die oben genannten Substanzen zu den natürlichen Nahrungsbestandteilen. Galacturonsäurepolymere stehen in naher Beziehung zu Pektinen, die in nahezu allen Pflanzenbestandteilen vorkommen. Die Verbindungen können oral als Granulate, Sirup oder Suspensionen verabreicht werden.

Zur Behandlung und Vorbeugung krankhafter Abläufe der Biomorphose, insbesondere des Lipidhaushaltes, wie Stoffwechselentgleisungen und Gefäßveränderungen bei Atherosklerose, diabetischen Gefäßveränderungen, Störungen der Netzhautdurchblutung, werden im Durchschnitt täglich ca. 10 bis 50 g des erfindungsgemäßen Wirkstoffs verabreicht. Sie werden zu den Hauptmahlzeiten eingenommen.

In den unten berichteten Versuchen wurde nachgewiesen, daß die α-D-Galacturonsäure, das Monomer des Pektin, eine hypocholesterinämische Wirkung im Serum zeigt. Galacturonsäure ist im Gegensatz zu Pektin gut wasserlöslich und kann deshalb, wie erwähnt, problemlos eingenommen werden. Die Behandlung einer Cholesterinämie mit α-D-Galacturonsäure ist relativ unproblematisch. Toxische Effekte sind nicht bekannt. Die α-D-Galacturonsäure ist ein Zucker, der im Pflanzenreich sehr häufig vorkommt. Durch acetalartige Verknüpfung der DEAE-Anionenaustauschergruppe an zwei der drei C-O-Gruppen des Pektinmonomers Galacturonsäure ließ sich die cholesterinsenkende Wirkung im Hamstermodell etwa verdoppeln.

Zu erwähnen wäre auch, daß anstelle der α-D-Galacturonsäure beispielsweise auch die β-D-Galacturonsäure gemäß der Erfindung zum Einsatz gelangen könnte. Letztere ist jedoch erheblich teurer, so daß in der Regel gemäß der Erfindung die α-D-Galacturonsäure zum Einsatz gelangen wird.

Die unten berichteten Versuche belegen eindeutig, daß die Einnahme von Galacturonsäure zu einem signifikanten Absenken der Cholesterin- und Lipidkonzentrationen führt, welche zumindest zum Teil für die Entstehung von Hyperlipidämie und/oder Atherosklerose verantwortlich sind.

Die unten dargestellten Versuchsergebnisse zeigen, daß alle hier untersuchten Substanzen, die α-D-Galacturonsäure enthalten, wirksam sind. Die Methylierung der Säuregruppe der Galacturonsäure beeinflußt die Wirkung der α-D-Galacturonsäure nur geringfügig. Die α-D-Galacturonsäure ist sowohl als Monomer als auch als Polymer in etwa gleichem Maße wirksam.

Auch durch die Fermentierung des Pektins entsteht hauptsächlich Galacturonsäure, so daß also fermentiertes Pektin eingesetzt werden kann.

Die Substitution einer Anionenaustauschergruppe steigert die Wirksamkeit jedoch nochmals deutlich. Diese Steigerung ist bedingt durch die Anionenaustauscherkapazität des tertiären Stickstoffes der DEAE-Seitengruppe. Eine Wirkungssteigerung wird mit Sicherheit auch von anderen, tertiären oder quartären Amine enthaltenden Anionenaustauschergruppen erzielt.

Es ist aus diesen Gründen offensichtlich, daß alle beanspruchten Substanzen auch als hypolipidämische und antiatherosklerotische Arzneimittel wirksam sind.

### Pharmakologische Prüfung

Galacturonsäure und deren Derivate sind in der Regel gut verträglich. Die LD 50 von Galacturonsäurepolymeren liegt weit über 10 g/kg bei Ratten, Hamstern und Kaninchen. Auch die anderen oben genannten Verbindungen weisen ähnlich gute Verträglichkeit auf. Durch ihre Verwandschaft bzw. Zugehörigkeit zu natürlichen Bestandteilen der Nahrung ist die Harmlosigkeit der Verbindungen sichergestellt.

### Präklinische Prüfung

### Versuch 1:

Je 20 männliche Goldhamster in 3 Gruppen wurden 4 Wochen mit einer Lipidämiediät (Hamsterhaltungsdiät und 2 % Cholesterin) gefüttert. Das Futter der beiden behandelten Gruppen wurde zusätzlich mit je 5 % Galacturonsäure bzw. DEAE-Poly-Galacturonsäure (DEAE = Diethylaminoetyl) angereichert.

Die aufgenommene Dosis lag zwischen 4 und 5 g/kg Körpergewicht und Tag.

Nach 4 Wochen wurden die Tiere geötet und die Serum- und Leber-Lipidkonzentration gemessen. Die Resultate der Analysen sind in Tabelle 1, 2 und 3 zusammengefaßt.

Galacturonsäure senkte nach vierwöchiger Behandlung im Serum die Konzentration von Gesamt-Cholesterin um 20 %, vom HDL-Cholesterin um 20 %, vom LDL-Cholesterin um 23 %, vom VLDL- und LDL-Cholesterin um 20 % und vom freien Cholesterin um 19 % signifikant (p ≦ 0.05). Außerdem senkte Galacturonsäure im Serum die Gesamt-Lipidkonzentration um 18 % und die Phospholipidkonzentration um 17 % signifikant. Ferner war noch die Gesamt-Lipidkonzentration in der Leber um 34 % signifikant erniedrigt.

DEAE-Poly-Galacturonsäure senkte nach vierwöchiger Behandlung im Serum die Konzentration von Gesamt-Cholesterin um 43 %, vom HDL-Cholesterin um 47 %, vom LDL-Cholesterin um 46 %, vom VLDL-Cholesterin um 24 % und vom VLDL- plus LDL-Cholesterin um 41 % signifikant im Vergleich zu den Kontrolltieren. Außerdem war noch die Gasamt-Lipidkonzentration um 37 % und die Phospholipidkonzentration um 41 % signifikant im Vergleich zu den Kontrolltieren gesenkt.

Die Leber-Cholesterinkonzentration war bei den mit DEAE-Poly-Galacturonsäure behandelten Tieren um 21 % und die Leber-Gesamt-Lipidkonzentration um 44 % signifikant gegenüber den Kontrolltieren gesenkt.

### Versuch 2:

Je 20 männliche Goldhamser in 3 Gruppen wurden 4 Wochen wie die in 1. beschriebenen Tiere behandelt. Gruppe 2 wurde mit 5 % Polygalacturonsäure, Gruppe 3 wurde mit 5 % Polygalacturonsäure-Methylester im Futter behandelt. Gruppe 1 war die Lipidämiekontrolle. Die Serum- und Leberlipidkonzentrationen nach der vierwöchigen Behandlung sind in Tabelle 4, 5, 6 zusammengefaßt.

Polygalacturonsäure senkte im Serum nach der vierwöchigen Behandlung gegenüber den Kontrolltieren das Gesamt-Cholesterin um 13 %, das HDL-Cholesterin um 15 % und das freie Cholesterin um 32 %, sowie die Gesamt-Lipidkonzentration um 14 % und die Phospholipidkonzentration um 19 % signifikant (p ≦ 0.05 Tabelle 4 und 5). Ferner senkte die Polygalacturonsäurebehandlung die Leber-Cholesterinkonzentration um 26 % und die Leber-Gesamtlipidkonzentration um 25 % signifikant.

Polygalacturonsäure-Methylester senkte nach vierwöchiger Behandlung im Serum die Konzentration des Gesamt- (-24 %), HDL-(-24 %), LDL- (-23 %), VLDL- (-44 %), VLDL- plus LDL-(-22 %) und des freien Cholesterins (-41 %) signifikant (Tabelle 4).

Ferner senkte Polygalacturonsäure-Methylester im Serum die Konzentration der Gesamtlipide um 18 % und der Phospholipide um 23 % signifikant (Tabelle 5). In der Leber senkte Polygalacturonsäure-Methylester die Cholesterinkonzentration um 29 % und die Gesamtlipidkonzentration um 32 % signifikant.

### Herstellung des fermentierten Pektins:

Vorgelegt wird ein 50 mMol NaH₂PO₄ Puffer pH 4,5, 60°C und 1 g Pektinase/l (Pektinase 5 S, Serva, Aspergillus niger, 0,66 U/mg). In diese Lösung wird über mehrere Stunden schrittweise Pektin eingerührt und bis zur Endkonzentration von ca. 500 g/l und pro kg Pektin wird 10 g Pektinase zugegeben. Der pH wird durch Zugabe von 5M NaOH auf pH 4,5 eingestellt.

**Tabelle 1**

| Cholesterinkonzentration im Hamsterserum nach vierwöchiger Behandlung (n = 20/Gruppe) Mittelwerte [mg/dl] ± Standardabweichung (SD) | | | |
|---|---|---|---|
| | Kontrolle | Galacturonsäure | DEAE-Polygalacturonsäure |
| Gesamt-Cholesterin | 340 | 271* | 193* |
| SD | ±53 | ±31 | ±40 |
| D | | -20 | -43 |
| HDL-Cholesterin | 148 | 118* | 79* |
| SD | ±23 | ±21 | ±13 |
| D | | -20 | -47 |
| LDL-Cholesterin | 151 | 117* | 82* |
| SD | ±42 | ±24 | ±24 |
| D | | -23 | -46 |
| VLDL-Cholesterin | 42 | 36 | 32* |
| SD | ±10 | ±12 | ± 8 |
| D | | -14 | -24 |
| VLDL- + LDL-Cholesterin | 192 | 153* | 114* |
| SD | ±47 | ±22 | ±30 |
| D | | -20 | -41 |
| Freies-Cholesterin | 118 | 96* | 103 |
| SD | ±28 | ±19 | ±20 |
| D | | -19 | -13 |
| D = Differenz in % bezogen auf die Kontrollgruppe | | | |

| | | | |
|---|---|---|---|
| * Signifikanz p ≦ 0.05 | | | |

**Tabelle 2**

| Lipidkonzentration im Hamsterserum nach vierwöchiger Behandlung (n = 20/Gruppe) Mittelwerte [mg/dl] ± Standardabweichung (SD) | | | |
|---|---|---|---|
| | Kontrolle | Galacturonsäure | DEAE-Polygalacturonsäure |
| Gesamtlipide | 1295 | 1060* | 819* |
| SD | ±177 | ±153 | ±135 |
| D | | -18 | -37 |
| Phospholipide | 389 | 322* | 228* |
| SD | ±48 | ±40 | ±31 |
| D | | -17 | -41 |
| Triglyceride | 202 | 216 | 188 |
| SD | ±76 | ±82 | ±55 |
| D | | + 7 | - 7 |
| D = Differenz in % bezogen auf die Kontrollgruppe | | | |

| | | | |
|---|---|---|---|
| * Signifikanz p ≦ 0.05 | | | |

**Tabelle 3**

| Leberlipidkonzentration (mg/g Feuchtgewicht) von Hamstern nach vierwöchiger Behandlung (n = 20/Gruppe) Mittelwerte [mg/g Feuchtgewicht] ± Standardabweichung (SD) | | | |
|---|---|---|---|
| | Kontrolle | Galacturonsäure | DEAE-Polygalacturonsäure |
| Cholesterin | 30 | 27 | 24* |
| SD | ± 3 | ± 6 | ± 8 |
| D | | - 9 | -21 |
| Gesamtlipide | 169 | 111* | 94* |
| SD | ±22 | ±25 | ±33 |
| D | | -34 | -44 |
| D = Differenz in % bezogen auf die Kontrollgruppe | | | |

| | | | |
|---|---|---|---|
| * Signifikanz p ≦ 0.05 | | | |

**Tabelle 4**

| Cholesterinkonzentration im Hamsterserum nach vierwöchiger Behandlung (n = 20/Gruppe) Mittelwerte [mg/dl] ± Standardabweichung (SD) | | | |
|---|---|---|---|
| | Kontrolle | Polygalacturonsäure Methylester | Polygalacturonsäure |
| Gesamt-Cholesterin | 414 | 314* | 361* |
| SD | ±54 | ±44 | ±49 |
| D | | -24 | -13 |
| HDL-Cholesterin | 175 | 133* | 149* |
| SD | ±26 | ±19 | ±23 |
| D | | -24 | -15 |
| LDL-Cholesterin | 199 | 153* | 163 |
| SD | ±63 | ±36 | ±38 |
| D | | -23 | -18 |
| VLDL-Cholesterin | 50 | 28* | 48 |
| SD | ±24 | ±12 | ±16 |
| D | | -44 | - 4 |
| VLDL- + LDL-Cholesterin | 231 | 181* | 212 |
| SD | ±47 | ±41 | ±42 |
| D | | -22 | - 8 |
| Freies-Cholesterin | 117 | 69* | 80* |
| SD | ±17 | ±10 | ±13 |
| D | | -41 | -32 |
| D = Differenz in % bezogen auf die Kontrollgruppe | | | |

| | | | |
|---|---|---|---|
| * Signifikanz p ≦ 0.05 | | | |

**Tabelle 5**

| Lipidkonzentration im Hamsterserum nach vierwöchiger Behandlung (n = 20/Gruppe) Mittelwerte [mg/dl] ± Standardabweichung (SD) | | | |
|---|---|---|---|
| | Kontrolle | Polygalacturonsäure Methylester | Polygalacturonsäure |
| Gesamtlipide | 1563 | 1286* | 1338* |
| SD | ±151 | ±181 | ±201 |
| D | | -18 | -14 |
| Phospholipide | 474 | 365* | 385* |
| SD | ±52 | ±44 | ±47 |
| D | | -23 | -19 |
| Triglyceride | 270 | 265 | 269 |
| SD | ±59 | ±80 | ±89 |
| D | | - 2 | - |
| D = Differenz in % bezogen auf die Kontrollgruppe | | | |

| | | | |
|---|---|---|---|
| * Signifikanz p ≦ 0.05 | | | |

**Tabelle 6**

| Leberlipidkonzentration (mg/g Feuchtgewicht) von Hamstern nach vierwöchiger Behandlung (n = 20/Gruppe) Mittelwerte [mg/g Feuchtgewicht] ± Standardabweichung (SD) | | | |
|---|---|---|---|
| | Kontrolle | Polygalcturonsäure Methylester | Polygalacturonsäure |
| Cholesterin | 30.0 | 21.3* | 22.1* |
| SD | ±3.8 | ±7.3 | ±4.9 |
| D | | -29 | -26 |
| Gesamtlipide | 89.4 | 60.4* | 67.4* |
| SD | ±11.6 | ±17.4 | ±23.0 |
| D | | -32 | -25 |
| D = Differenz in % bezogen auf die Kontrollgruppe | | | |

| | | | |
|---|---|---|---|
| * Signifikanz p ≦ 0.05 | | | |

## Patentansprüche (Patentansprüche für folgende(n) Vertragsstaat(en): AT, BE, CH, DE, DK, FR, GB, IT, LI, LU, NL, SE)

1. Verwendung von Galacturonsäure, insbesondere der α-D Galacturonsäure der allgemeinen Formel 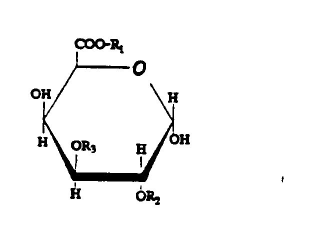 wobei
R₁ = H,
R₂ = H und
R₃ = H,
zur Herstellung eines Arzneimittels zur Propylaxe und Therapie von Hyperlipidämie und/oder Atherosklerose.

2. Verwendung von Polymeren der Galacturonsäure, insbesondere der α-Galacturonsäure der allgemeinen Formel, 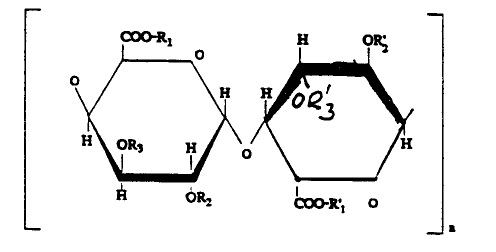 wobei
R₁ = H R'₁ = H
R₂ = H und R'₂ = H
R₃ = H R'₃ = H und
n eine ganze Zahl ist zu dem in Anspruch 1 angegebenen Zweck.

3. Verwendung von Methylester der Galacturonsäure und deren Polymere, insbesondere der α-D Galacturonsäure zu dem in Anspruch 1 angegebenen Zweck, wobei
wobei
R₁ = CH₃ R'₁ = CH₃ oder H,
R₂ = H R'₂ = H
R₃ = H, R'₃ = H.

4. Verwendung zu dem in Anspruch 1 angegebenen Zweck von tertiäre oder quartäre Amin-Anionenaustauscher enthaltende Ester, Ether und/oder Acetale der Galacturonsäure, insbesondere der α-D Galacturonsäure sowie deren Polymere, wobei
im Falle der Ester
R₁ = - CH₂ - R₄ R'1 = R₁ oder H
R₂ = H R'2 = H
R₃ = H R'3 = H,
im Falle der Ether
R₁ = H oder CH₃ R'1 = R₁
R₂ oder R₃ = - CH₂ - R₄ R'2 = R₂ oder H
R'3 = R₃ oder H,
im Falle der Acetale 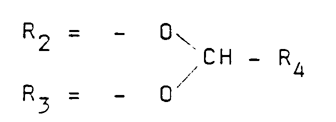 die Amin-Anionenaustauscher in der allgemeinen Formel durch 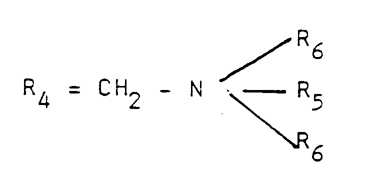 gebildet sind,
und wobei im Falle des tertiären Amins
R₅ = H
und im Falle des quartären Amins
R₅ = CH₃ oder
R₅ = [CH₂] ₘ - CH₃ oder
R₅ = [CH₂] ₘ CH (OH) - CH₃
R₆ = [CH₂] ₘ - CH₃, wobei
m = eine ganze Zahl von 1 bis 5 ist.

5. Verwendung von fermentiertem Pektin zu dem in Anspruch 1 angegebenen Zweck.

6. Oral verabreichbares Arzneimittel zur Behandlung von Hyperlipidämie und/oder Atherosklerose, dadurch gekennzeichnet, daß es als Wirkstoff mindestens eine Verbindung wie in Anspruch 1 bis 5 beschrieben enthält.

## Patentansprüche (Patentansprüche für folgende(n) Vertragsstaat(en): ES, GR)

1. Verwendung von Galacturonsäure, insbesondere der α-D Galacturonsäure der allgemeinen Formel 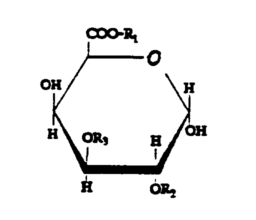 wobei
R₁ = H,
R₂ = H und
R₃ = H,
zur Herstellung eines Arzneimittels zur Propylaxe und Therapie von Hyperlipidämie und/oder Atherosklerose.

2. Verwendung von Polymeren der Galacturonsäure, insbesondere der α-Galacturonsäure der allgemeinen Formel, 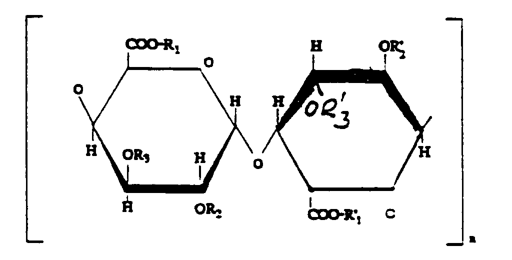 wobei
R₁ = H R'₁ = H
R₂ = H und R'₂ = H
R₃ = H R'₃ = H und
n eine ganze Zahl ist zu dem in Anspruch 1 angegebenen Zweck.

3. Verwendung von Methylester der Galacturonsäure und deren Polymere, insbesondere der α-D Galacturonsäure zu dem in Anspruch 1 angegebenen Zweck, wobei
wobei
R₁ = CH₃ R'₁ = CH₃ oder H,
R₂ = H R'₂ = H
R₃ = H, R'₃ = H.

4. Verwendung zu dem in Anspruch 1 angegebenen Zweck von tertiäre oder quartäre Amin-Anionenaustauscher enthaltende Ester, Ether und/oder Acetale der Galacturonsäure, insbesondere der α-D Galacturonsäure sowie deren Polymere, wobei
im Falle der Ester
R₁ = - CH₂ - R₄ R'1 = R₁ oder H
R₂ = H R'2 = H
R₃ = H R'3 = H,
im Falle der Ether
R₁ = H oder CH₃ R'1 = R₁
R₂ oder R₃ = - CH₂ - R₄ R'2 = R₂ oder H
R'3 = R₃ oder H,
im Falle der Acetale 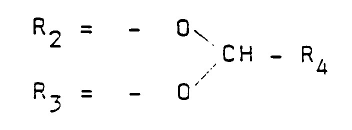 die Amin-Anionenaustauscher in der allgemeinen Formel durch 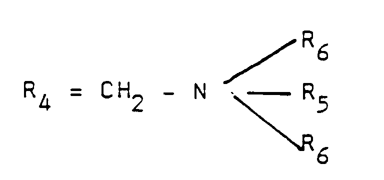 gebildet sind,
und wobei im Falle des tertiären Amins
R₅ = H
und im Falle des quartären Amins
R₅ = CH₃ oder
R₅ = [CH₂] ₘ - CH₃ oder
R₅ = [CH₂] ₘ CH (OH) - CH₃
R₆ = [CH₂] ₘ - CH₃, wobei
m = eine ganze Zahl von 1 bis 5 ist.

5. Verwendung von fermentiertem Pektin zu dem in Anspruch 1 angegebenen Zweck.

6. Verfahren zur Herstellung eines oral verabreichbaren Arzneimittels zur Behandlung von Hyperlipidämie und/oder Atherosklerose, dadurch gekennzeichnet, daß ihm als Wirkstoff mindestens eine Verbindung wie in Anspruch 1 bis 5 beschrieben zugegeben wird.

## Claims (Claims for the following Contracting State(s): AT, BE, CH, DE, DK, FR, GB, IT, LI, LU, NL, SE)

1. Use of galacturonic acid, especially of α-D-galacturonic acid of the general formula 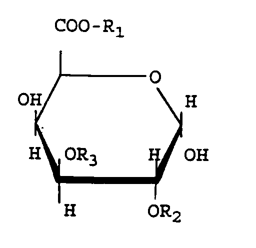 where
R₁ = H,
R₂ = H and
R₃ = H,
for the preparation of a medicament for the prophylaxis and therapy of hyperlipidaemia and/or atherosclerosis.

2. Use of polymers of galacturonic acid, especially of α-galacturonic acid of the general formula 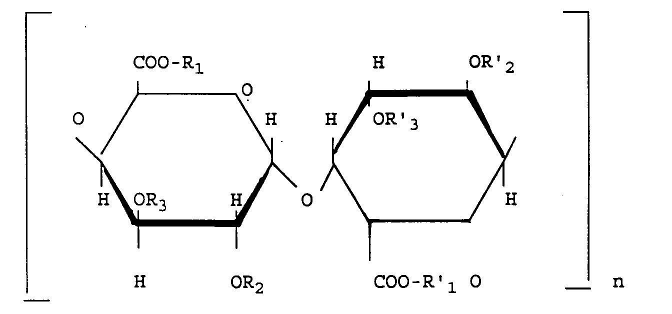 where
R₁ = H R'₁ = H
R₂ = H and R'₂ = H
R₃ = H R'₃ = H and
n is an integer, for the purpose indicated in Claim 1.

3. Use of methyl enters of galacturonic acid and polymers thereof, especially of α-D-galacturonic acid for the purpose indicated in Claim 1,
where
R₁ = CH₃ R'₁ = CH₃ or H,
R₂ = H R'₂ = H
R₃ = H R'₃ = H.

4. Use for the purpose indicated in Claim 1 of enters, ethers and/or acetals, containing tertiary or quaternary amine anion exchangers, of galacturonic acid, especially of α-D-galacturonic acid and polymers thereof, where
in the case of the esters
R₁ = -CH₂-R₄ R'1 = R₁ or H
R₂ = H R'2 = H
R₃ = H R'3 = H,
in the case of the ethers
R₁ = H or CH₃ R'1 = R₁
R₂ or R₃ = -CH₂-R₄ R'2 = R₂ or H
R'3 = R₃ or H,
in the case of the acetals 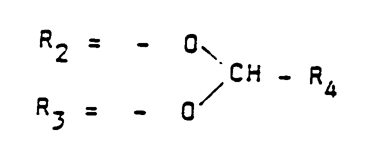 the amine anion exchangers in the general formula are formed by 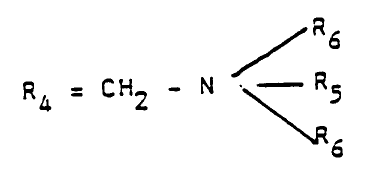 and where in the case of the tertiary amine
R₅ = H
and in the case of the quaternary amine
R₅ = CH₃ or
R₅ = [CH₂]ₘ-CH₃ or
R₅ = [CH₂]ₘ CH(OH)-CH₃
R₆ = [CH₂]ₘ-CH₃, where
m is an integer from 1 to 5.

5. Use of fermented pectin for the purpose indicated in Claim 1.

6. Medicament which can be administered orally for the treatment of hyperlipidaemia and/or atherosclerosis characterized in that it contains at least one compound according to Claims 1 to 5 as active substance.

## Claims (Claims for the following Contracting State(s): ES, GR)

1. Use of galacturonic acid, especially of α-D-galacturonic acid of the general formula 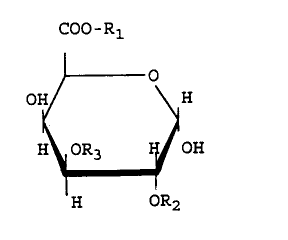 where
R₁ = H,
R₂ = H and
R₃ = H,
for the preparation of a medicament for the prophylaxis and therapy of hyperlipidaemia and/or atherosclerosis.

2. Use of polymers of galacturonic acid, especially of α-galacturonic acid of the general formula 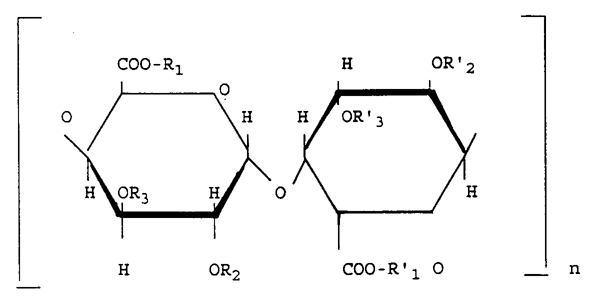 where
R₁ = H R'₁ = H
R₂ = H and R'₂ = H
R₃ = H R'₃ = H and
n is an integer, for the purpose indicated in Claim 1.

3. Use of methyl esters of galacturonic acid and polymers thereof, especially of α-D-galacturonic acid for the purpose indicated in Claim 1,
where
R₁ = CH₃ R'₁ = CH₃ or H,
R₂ = H R'₂ = H
R₃ = H R'₃ = H.

4. Use for the purpose indicated in Claim 1 of esters, ethers and/or acetals, containing tertiary or quaternary amine anion exchangers, of galacturonic acid, especially of α-D-galacturonic acid and polymers thereof,
where
in the case of the esters
R₁ = -CH₂-R₄ R'1 = R₁ or H
R₂ = H R'2 = H
R₃ = H R'3 = H,
in the case of the ethers
R₁ = H or CH₃ R'1 = R₁
R₂ or R₃ = -CH₂-R₄ R'2 = R₂ or H
R'3 = R₃ or H,
in the case of the acetals 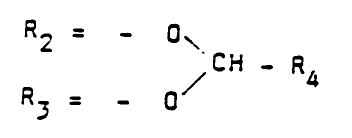 the amine anion exchangers in the general formula are formed by 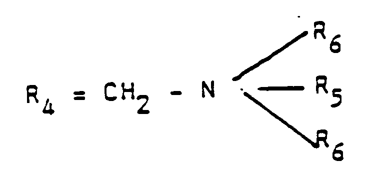 and where in the case of the tertiary amine
R₅ = H
and in the case of the quaternary amine
R₅ = CH₃ or
R₅ = [CH₂]ₘ-CH₃ or
R₅ = [CH₂]ₘ CH(OH)-CH₃
R₆ = [CH₂]ₘ-CH₃, where
m is an integer from 1 to 5.

5. Use of fermented pectin for the purpose indicated in Claim 1.

6. Process for the preparation of a medicament which can be administered orally for the treatment of hyperlipidaemia and/or atherosclerosis characterized in adding at least one compound according to Claims 1 to 5 as active substance.

## Revendications (Revendications pour l'(les) Etat(s) contractant(s) suivant(s): AT, BE, CH, DE, DK, FR, GB, IT, LI, LU, NL, SE)

1. Utilisation de l'acide galacturonique, en particulier de l'acide α-D-galacturonique de formule générale: 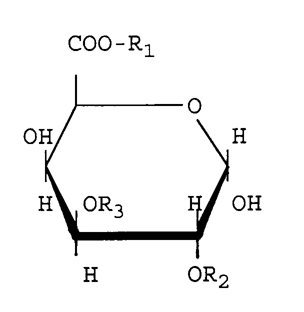 dans laquelle:
R₁ = H,
R₂ = H
et
R₃ = H,
pour la production d'un médicament pour la prophylaxie et la thérapeutique de l'hyperlipidémie et/ou de l'athérosclérose.

2. Utilisation de polymères d'acide galacturonique, en particulier de l'acide α-D-galacturonique, de formule générale: 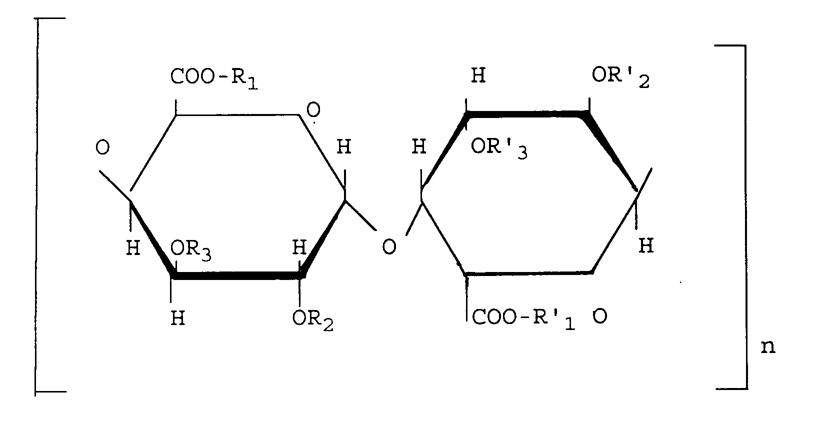 dans laquelle:
R₁ = H R'₁ = H
R₂ = H R'₂ = H
R₃ = H R'₃ = H et
n est un nombre entier,
pour le but mentionné dans la revendication 1.

3. Utilisation de l'ester méthylique de l'acide galacturonique et de ses polymères en particulier de l'acide α-D-galacturonique, pour le but mentionné dans la revendication 1, dans laquelle,
R₁ = CH₃ R'₁ = CH₃ ou H,
R₂ = H R'₂ = H
R₃ = H R'₃ = H

4. Utilisation pour le but indiqué à la revendication 1, d'esters d'éthers et/ou d'acétals d'acide galacturonique, en particulier de l'acide α-D-galacturonique ainsi que de ses polymères, contenant des échangeurs d'anions et d'amine tertiaires ou quaternaires, dans lesquels:
dans le cas des esters :
R₁ = -CH₂ - R₄ R'1 = R₁ ou H
R₂ = H R'2 = H
R₃ = H R'3 = H,
dans le cas des éthers :
R₁ = H ou CH₃ R'1 = R₁
R₂ ou R₃ = -CH₂ - R₄ R'2 = R₂ ou H
R'3 = R₃ ou H,
dans le cas des acétals : 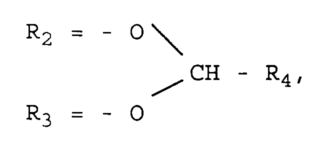 les échangeurs d'anions et d'amine dans la formule générale sont formés par : 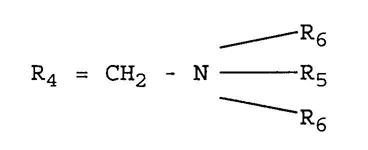 et dans lesquels dans le cas de l'amine tertiaire :
R₅ = H
et dans le cas de l'amine quaternaire
R₅ = CH₃ ou
R₅ = [CH₂]ₘ - CH₃ ou
R₅ = [CH₂]ₘ CH (OH) - CH₃
R₆ = [CH₂]ₘ - CH₃
dans laquelle m = un nombre entier de 1 à 5.

5. Utilisation de pectine fermentée pour le but indiqué à la revendication 1.

6. Médicament administrable oralement pour le traitement de l'hyperlipidémie et/ou de l'athérosclérose, caractérisé en ce qu'il contient en tant que principe actif, au moins un composé comme décrit dans les revendications 1 à 5.

## Revendications (Revendications pour l'(les) Etat(s) contractant(s) suivant(s): ES, GR)

1. Utilisation de l'acide galacturonique, en particulier de l'acide α-D-galacturonique de formule générale: 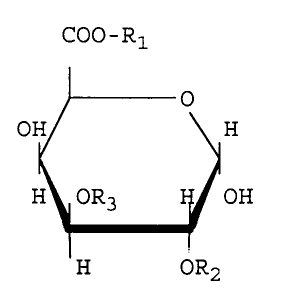 dans laquelle:
R₁ = H,
R₂ = H
et
R₃ = H,
en vue de la production d'un médicament pour la prophylaxie et la thérapeutique de l'hyperlipidémie et/ou de l'athérosclérose.

2. Utilisation de polymères d'acide galacturonique, en particulier de l'acide α-D-galacturonique de formule générale: 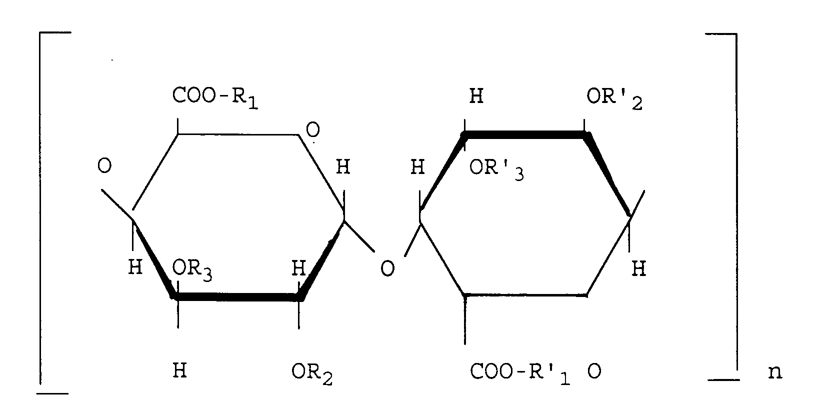 dans laquelle:
R₁ = H R'₁ = H
R₂ = H et R'₂ = H
R₃ = H R'₃ = H
n est un nombre entier,
pour le but mentionné dans la revendication 1.

3. Utilisation de l'ester méthylique de l'acide galacturonique et de ses polymères en particulier de l'acide α-D-galacturonique pour le but mentionné dans la revendication 1, dans lequel,
R₁ = CH₃ R'₁ = CH₃ ou H,
R₂ = H R'₂ = H
R₃ = H R'₃ = H

4. Utilisation pour le but indiqué à la revendication 1, d'esters, d'éthers et/ou d'acétals d'acide galacturonique, en particulier de l'acide α-D-galacturonique ainsi que de ses polymères contenant des échangeurs d'anions et d'amine tertiaires et quadernaires, dans lesquels:
dans le cas des esters :
R₁ = CH₂ - R₄ R'1 = R₁ ou H
R₂ = H R'2 = H
R₃ = H R'3 = H,
dans le cas des éthers :
R₁ = H ou CH₃ R'1 = R₁
R₂ ou R₃ = -CH₂ - R₄ R'2 = R₂ ou H
R'3 = R₃ ou H,
dans le cas des acétals : 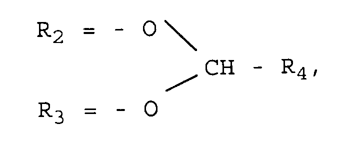 les échangeurs d'anions amine dans la formule générale sont formés par : 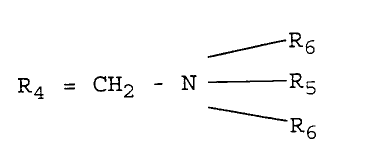 et dans lesquels dans le cas de l'amine tertiaire :
R₅ = H
et dans le cas de l'amine quaternaire
R₅ = CH₃ ou
R₅ = [CH₂]ₘ - CH₃ ou
R₅ = [CH₂]ₘ CH (OH) - CH₃
R₆ = [CH₂]ₘ - CH₃
dans laquelle m = un nombre entier de 1 à 5.

5. Utilisation de pectine fermentée pour le but indiqué à la revendication 1.

6. Procédé d'obtention d'un médicament administrable oralement pour le traitement de l'hyperlipidémie et/ou de l'athérosclérose, caractérisé en ce qu'il contient en tant que principe actif, au moins un composé comme décrit dans les revendications 1 à 5.
